# EUROPEAN PATENT APPLICATION

(11) **EP 1 266 633 A2**
(43) Date of publication of application: **18.12.2002**
(21) Application number: 02254155.1
(22) Date of filing: 14.06.2002
(51) Int. Cl.: A61B 17/64

(54) **Device for immobilising bones of a patient during surgery**

(30) Priority: 15.06.2001 GB 0114659
(71) Applicant: Finsbury (Development) Limited, Leatherhead, Surrey KT22 0BA (GB)
(72) Inventor: Wozencroft, Robert Michael, Epsom, Surrey KT19 8SS (GB)
(74) Representative: Eyles, Christopher Thomas

(57) **Abstract**

A device is described for immobilising bones of a patient requiring surgery or treatment. The device comprises a pair of substantially vertical first members (6) and at least one second member (7) extending between and bracing the first members (6), and support means (8,11,12) movably mounted on at least one of the first members (6) so as to permit adjustment of the height of the support means (8,11,12). A series of first cantilever members (17,18,19) and a series of second cantilever members (20,21,22). Each first cantilever member (17,18,19) is adapted to be supported at one end (65) by the support means (8,11,12). First bone engagement means (23) is mounted at and supported by the other ends (69) of the first cantilever members (17,18,19) for engagement with a surgically exposed first bone (F). Each second cantilever member (20,21,22) is adapted to be supported at one end (65) by the support means (8,11,12). Second bone engagement means (24) is mounted at and supported by the other ends (69) of the second cantilever members (20, 21, 22) for engagement with a surgically exposed second bone (T).

## Description

This invention relates to a bone immobilising device for immobilising bones of the patient during surgery, for example during robot assisted surgery, or treatment.

Surgery to implant a prosthesis in a joint requires the highest level of precision to ensure that the prosthesis is correctly positioned. Inaccurate positioning may result in complications occurring which will require further surgery for correction.

In recent years advances in both computer software and medical hardware engineering have occurred such that the use of surgical robots to assist in orthopaedic operations is now possible. The software controlling the robot can be programmed to cut only within a precisely defined area, thus preventing the accidental damage of healthy bone and tissue which may occur when the procedure is performed manually. Inaccuracies in the positioning of the prosthesis are said to be avoided as a result of the greater accuracy in cutting achieved by the robot.

In a pre-operative scan of the patient a computer model is built up of the bone or bones upon which the robot is to be used. In the case of a knee replacement operation, for example, the bones would be the femur, the tibia, the fibula and the patella. Such a computer model of the relevant bones in their respective positions can be built up from a pre-operative magnetic resonance imaging (MRI) scan or a computed tomography (CT) scan by reassembling from the two dimensional slices thus obtained a three dimensional model. In the case of an operation on a patient's knee, attention is paid to the whole leg in order to help achieve overall leg alignment when positioning the tibial and femoral implants. Models of the tibial and femoral implants are also loaded into the memory of the computer. These computer simulated models enable pre-operative planning, including positioning the implants in the bone or bones and defining of the bone cuts. The parameters within the computer software can then be used to control the robot in making the actual bone cuts and to show progress "on screen" in real time in the course of the operation. Adaptations can be made to the planned procedure during the actual operation depending on what is encountered.

This procedure is only possible when the position of the robot and the position of the real bones are known in the software. Effectively the bone models and real bones must be married together for the duration of the operation. The process to achieve this is known as registration and for the present purposes it is only necessary to describe registration of the bones, since it is assumed that the position of the robot is already known. To register the bones, the joint is exposed with the normal surgical approach. An electronic probe is used to touch on the exposed surface of the bone in several positions. This is confined to the exposed area. The approximate position of each point in turn will be indicated on screen. After several points are registered, the precise position of the real bone is deduced by the computer which already has the details of its shape present in its memory. This process is repeated, as necessary, for each bone involved in the surgical procedure.

Prior to registration it is essential that the bones are immobilised. After registration they should remain in that same position (i.e. stationary) for the duration of the bone cutting shaping procedure. If movement occurs, reregistration is required. However, if it is undetected, movement will seriously compromise the outcome of the procedure. Other systems exist where movement of the bones may be tracked after registration by electro-magnetic or optical sensors attached to the bone. These are generally referred to as "navigation systems" but they do not utilise a robot, preferring more traditional techniques for cutting the bone. A movement tolerance is introduced thus reducing accuracy. However, the robot system used for such operations as knee replacement relies on static holding of the bones.

In one current bone fixing device for use during knee surgery, a framework comprising a pair of generally U-shaped frames, each comprising a pair of uprights and a crosspiece, is secured with the frames in inverted position by their uprights to the standard rail system provided along each side of the operating table so that the crosspiece of each frame straddles the operating area. The crosspieces are in turn adjustably secured to each other by bracing members to which clamping members can be adjustably secured for clamping the tibia and femur after these have been exposed in the initial part of the operation. Thus, using this known device, the patient's knee is suspended from a secure bridge extending above the area of operation. Further stability can be achieved by providing a foot and lower leg rest around the area of the patient's heel so as to afford support to the lower limb.

A drawback of using this arrangement is that the apparatus is complex to set up and, due to the bulky nature of the device, at least part of the surgeon's critical working area may be obstructed. Furthermore, there may be difficulties in ensuring the sterility of all parts of the framework, thus increasing the danger of contamination. Moreover the bulky nature of the framework increases the chances of the scrubbed and sterile surgeon touching non-sterile parts while operating.

Therefore, there is a need in the art for a bone clamping device which securely fixes the surgically exposed bones of a joint, such as a knee joint, thus preventing movement of the joint and which does not obstruct the critical working area of the surgeon. Further, there is a need for a bone clamping device which minimises the possibility of contamination of the sterile area by non-sterile parts. Still further, there is a need in the art for a bone clamping device which can be set up quickly and easily.

According to the present invention there is provided a device for immobilising bones of a patient requiring surgery or treatment, the device comprising:
substantially vertical frame means including a pair of substantially vertical first members and at least one second member extending between and bracing the first members;
support means movably mounted on at least one of the first members so as to permit adjustment of the height of the support means and arranged to be securable to the at least one first member;
a series of first cantilever members each having a proximal end and a distal end, each first cantilever member being adapted to be supported at the proximal end thereof by the support means and to extend in cantilever fashion therefrom;
first bone engagement means mounted at and supported by the distal ends of the first cantilever members for engagement with a surgically exposed first bone of a patient for securing the first bone in a desired position;
a series of second cantilever members each having a proximal end and a distal end, each second cantilever member being adapted to be supported at the proximal end thereof by the support means and to extend in cantilever fashion therefrom; and
second bone engagement means mounted at and supported by the distal ends of the second cantilever members for engagement with a surgically exposed second bone of the patient for securing the second bone in a desired position.

In a preferred device the frame comprises a pair of second members which are integral with the first members and extend substantially horizontally therebetween.

Preferably the support means comprises a main support member extending between the first members and adapted to be movable on and adjustably securable to the first members and at least one subsidiary support member adapted to be movable on and adjustably securable to a respective first member and to be pivotable with respect thereto in a substantially horizontal plane. In such a device the at least one support member desirably further includes a pair of subsidiary support members each adapted to be movable on and pivotable in a substantially horizontal plane about a respective first member and to be adjustably securable thereto.

Although there may be more than three first cantilever members, e.g. 4, 5 or 6, it will normally be preferred that there are three first cantilever members. Thus in a particularly preferred from of device:
(i) the support means comprises:
   a main support member extending between the first members and adapted to be vertically movable on and adjustably securable to the first members; and
   a pair of subsidiary support members each adapted to be vertically movable on and pivotable in a substantially horizontal plane about a respective first member and to be adjustably securable thereto; and
(ii) each first cantilever member is supported at its proximal end by a respective one of the support members.

Similarly, although there may be 4, 5 or 6 or more second cantilever members, it will normally suffice for there to be three second cantilever members. Hence in the device
(i) the support means may comprise:
   a main support member extending between the first vertical members and adapted to be vertically movable on and adjustably securable to the first members; and
   a pair of subsidiary support members each adapted to be vertically movable on and pivotable in a substantially horizontal plane about a respective first member and to be adjustably securable thereto; and
(ii) each second cantilever member may be supported at its proximal end by a respective one of the support members.

Conveniently the substantially vertical frame means is adapted for securement to a rail extending along one side of an operating table. In this case each of the first members of the substantially vertical frame means may be provided with a respective foot adapted to engage with the rail and to be lockable thereto.

In one embodiment of the device the first bone engagement means is adapted to be engageable with a surgically exposed femur of the patient and the second bone engagement means is adapted to be engageable with a surgically exposed tibia of the patient. In this case the first bone engagement means may comprise a femoral gripping means comprising a pair of gripping jaw members pivoted one to another, each provided at its free end with femur contact means for contact with the femur, and adjustment means for closing the gripping jaws to cause the femur contact means to contact with and grip the femur and for opening the gripping jaws to release the femur. The femur contact means may thus comprise a plurality of self adjusting feet for contact with the surgically exposed femur of the patient. Alternatively the femur contact means may comprise a toggle mounted foot for contact with the surgically exposed femur of the patient.

The second bone engagement means may accordingly comprise a tibial attachment member adapted to be secured by means of pins or screws to a surgically exposed tibia of the patient.

Preferably the first cantilever members and/or the second cantilever members are adjustable in length.

In a preferred design the support means is provided with a plurality of downwardly extending vertical holes or recesses. In this design the proximal end of each first cantilever member and/or each second cantilever member can be provided with a spigot adapted for receipt in one of the vertical holes or recesses. Similarly the distal end of each first cantilever member and/or each second cantilever member can be provided with a spigot adapted for engagement with the first bone engagement means.

Preferably the first and second cantilever members are each adjustable in length. Thus the first and second cantilever members may each comprise an elongate outer tubular member and an elongate inner member adapted to slide telescopically in the outer tubular member, and locking means for locking the elongate inner member and the elongate outer tubular member one to another.

At least one cantilever member selected from the first cantilever members and the second cantilever members can be provided with a movement detector, which is preferably arranged to produce an alarm signal upon movement of the at least one cantilever member.

In order that the invention may be clearly understood and readily carried into effect, a preferred embodiment thereof will now be described, by way of example only, with reference to the accompanying drawings, wherein:
Figure 1 is a perspective view of a bone clamping device in accordance with the invention in position during a surgical procedure for implantation in a patient's knee of a knee prosthesis;
Figure 2 is a perspective view on an enlarged scale of the surgically exposed area of the patient's knee showing in more detail the femur clamp and also the tibial attachment member forming part of the device of Figure 1;
Figure 3 is a perspective view of the femur clamp of Figure 2;
Figure 4 is a further enlarged perspective view of one of the jaws of the femur clamp of Figures 1 to 3;
Figure 5 is a perspective view from above of a tibial attachment member forming part of the bone clamping device of Figure 1;
Figure 6 is a perspective view from below of the tibial attachment member of Figure 5;
Figure 7 is a further perspective view on an enlarged scale of a part of the tibial attachment member of Figures 5 and 6;
Figure 8 is a perspective view of the frame of the bone clamping device of Figure 1 with its cantilever arms in the folded storage position;
Figure 9 is a similar perspective view of the frame of Figure 10 with the cantilever arms in their open operative positions;
Figure 10 is a perspective view of one of the telescopic cantilever members forming part of the bone clamping device of Figure 1 in its extended condition;
Figure 11 is a perspective view on an enlarged scale of some of the parts of the telescopic cantilever member of Figures 10 in disassembled form;
Figure 12 is a further perspective view on an enlarged scale of the proximal end of the telescopic cantilever member of Figures 10 and 11;
Figure 13 is a detail perspective view of the distal end of the telescopic cantilever member of Figures 10 to 12;
Figure 14 is a perspective view of a modified form of femoral clamp; and
Figure 15 is a perspective view of a further modified form of femur clamp.

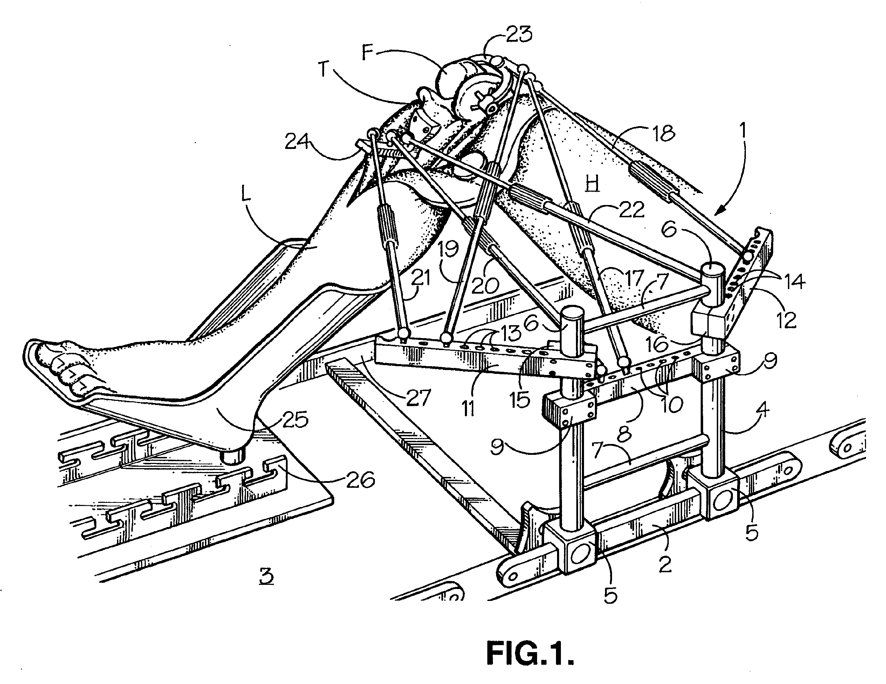

Referring to the drawings, and to Figure 1 in particular, a bone clamping device 1 in accordance with the invention is clamped on a rail 2 which extends along one side of an operating table 3. Device 1 comprises a frame 4 which, in use, extends substantially vertically above rail 2 along which it can be slid and secured in place by means of adjustable clamping feet 5. Frame 4 includes a pair of vertical first members 6 connected together by means of crossbars 7. A generally horizontal main support member 8 is slidably mounted on vertical members 6 and can be secured in position thereon by means of adjustable clamps 9. In this way the vertical position of main support member 8 with respect to the rail 2 can be varied at will by the surgeon. Support member 8 is further provided with a plurality of vertical holes of recesses 10. Also adjustably mounted on each of the vertical members 6 is a respective pivotable arm 11, 12, each of which is also provided with a series of vertical holes or recesses 13, 14. Arms 11, 12 can each be pivoted in a horizontal plane about a respective vertical member 6 and can be secured in position on its vertical member 6 by means of a clamp 15 or 16.

Support member 8 and arms 11, 12 provide support for the proximal ends of first cantilever members 17, 18, 19 and for the proximal ends of second cantilever members 20, 21, 22.

At their distal ends first cantilever members 17, 18, 19 provide support for a femur clamp 23, while the distal ends of second cantilever members 20, 21, 22 provide support for a tibial attachment member 24. Femur clamp 23 is adapted to grip, as will be explained in more detail below, a surgically exposed femur F of a thigh H of a patient, while tibial attachment member 24 is adapted to be secured, as will be described further hereinafter, to a surgically exposed tibia T of the lower leg L of the patient.

A lower leg support 25 can be used to support the heel of the patient's lower leg L which is arranged to engage in known manner with a rack 26 affixed to the operating table 3.

Reference numeral 27 indicates a rail on the farther side of the operating table, which will be the side of the operating table at which the surgeon stands during the course of the knee prosthesis implantation operation. Although as illustrated the device 1 is fitted to the rail 2 at the patient's left side for the surgeon to stand at the far side as illustrated of the operating table 3, the device 1 can equally well be fitted to the rail 27 instead, if the surgeon prefers to operate from the near side of the operating table 3 as illustrated.

It will thus be seen that device 1 supports the bones of the patient's knee during the knee prosthesis implantation operation from one side only of the operating table. Moreover the cantilever members 17 to 22 lie substantially at or below the operating area and not above it. Furthermore the device 1 securely fixes the patient's knee joint and enables maintenance of the fixed position throughout the surgical procedure. Because of its cantilevered design device 1 does not obstruct access to the operating area of the patient's exposed femur and tibia and permits the surgeon an essentially unobstructed view of the operating area.

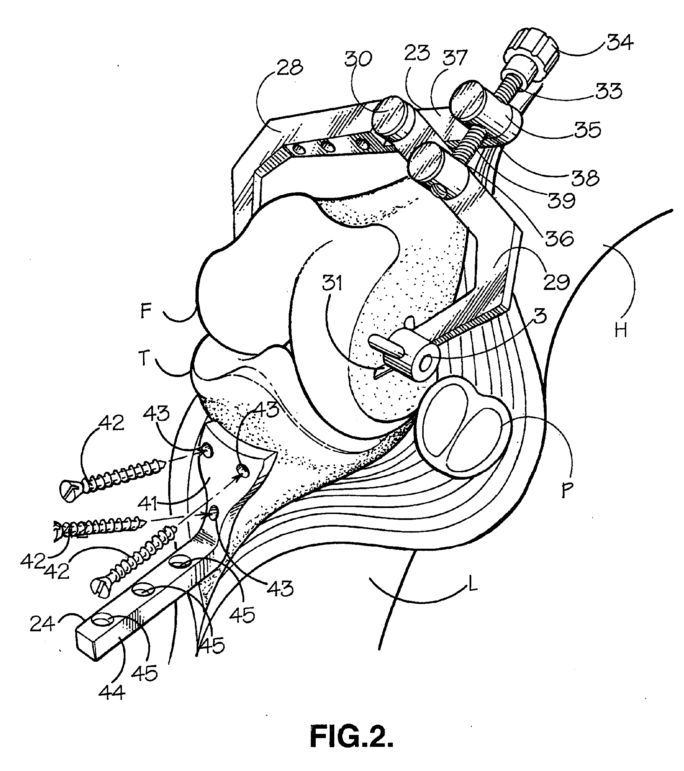

Figure 2 shows the femur clamp 23 and the tibial attachment member 24 in more detail as well as their attachment to the femur F and tibia T respectively. These bones, i.e. the femur F and tibia T have been surgically exposed in the preliminary part of the knee prosthesis implantation operation, with the patella P having been displaced to one side of the patient's knee joint. For the sake of simplicity the first and second cantilever members 17 to 22 have been omitted from Figure 2.

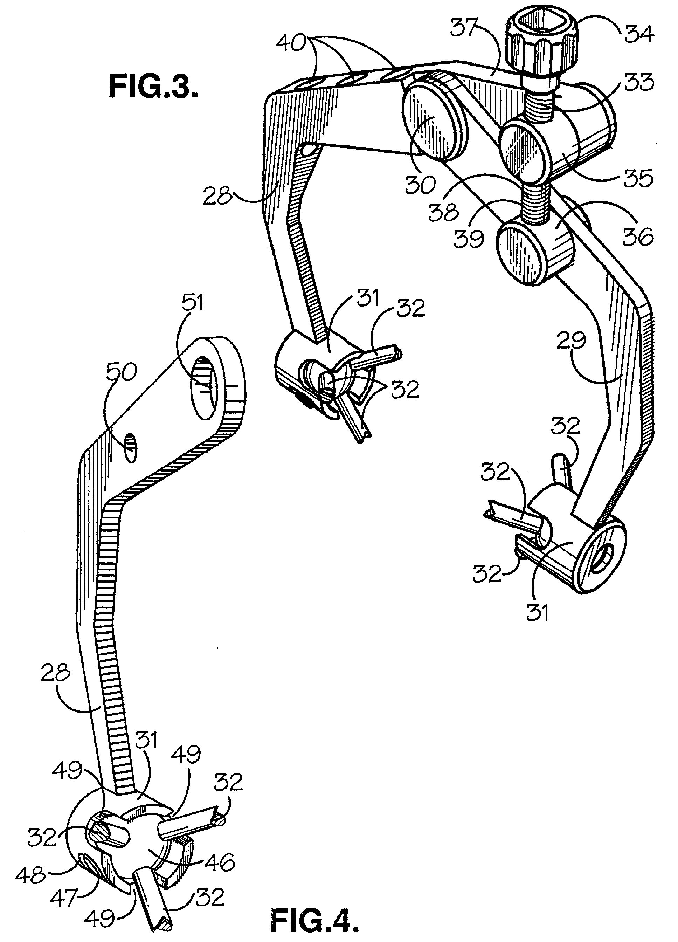

As can perhaps be seen best from Figure 3, femur clamp 23 has a pair of jaws 28, 29 connected one to another by pivot 30. At their free ends each jaw 28, 29 carries a socket 31 forming part of a self-adjusting foot provided with three prongs 32. The femur clamp 23 can be opened and closed by means of an adjustment screw 33 which carries a knurled adjustment knob 34 and is journalled in appropriate threaded bores in stubs 35, 36. As can be seen from Figures 2 and 3, stub 35 is pivotably secured to an extension 37 of jaw 28 while stub 36 is pivotably secured to jaw 29. Adjustment screw 33 carries portions 38, 39 of oppositely handed screw thread. Hence by turning knurled knob clockwise the jaws 28, 29 can be closed to grip femur F in a pincer-like motion, while rotating the knurled knob 34 anticlockwise the jaws 28, 29 can be made to open to release femur F. Jaw 28 is provided with three sockets 40 (see Figure 3) for a purpose which will be described hereinbelow.

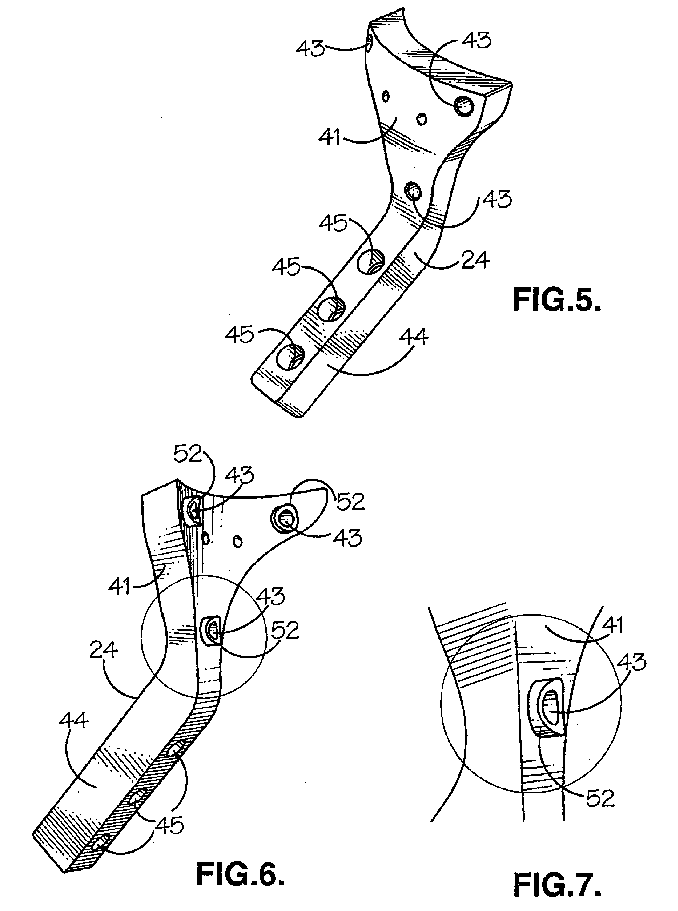

Tibial attachment member 24 is shown in more detail in Figures 5 to 7. It comprises a flange portion 41 which can be offered up to the patient's exposed tibia T and secured thereto by means of screws 42 which are inserted through corresponding screw holes 43. An extension 44 is set at an angle to flange portion 41 and is provided with a number of holes 45 for a purpose which will be described further below.

Referring now to Figure 4, it can be seen that the prongs 32 of the feet of femur clamp 23 are affixed to a ball member 46 which is received in socket 31 and is retained therein by means of a screw 47 which is engaged in a corresponding threaded bore in ball member 46. A slot 48 is provided in socket 31 in which the head of screw 47 can slide to permit limited motion of ball member 46 relative to socket 31. Open ended slots 49 receive prongs 32 and allow these to adopt an appropriate position, as femur clamp 23 is tightened, to grip the patient's femur F. Bore 50 is provided for receipt of pivot 30, while bore 51 receives stub 35.

The tibial attachment member 24 is shown in greater detail in Figures 5 to 7. As can be seen from Figures 6 and 7 there are ring shaped projections 52 surrounding each of screw holes 43 on the side of tibial attachment member 24 that is presented to the patient's tibia T. These help to locate the tibial attachment member 24 on the patient's tibia T and to space the main part of the tibial attachment member 24 from the tibia T.

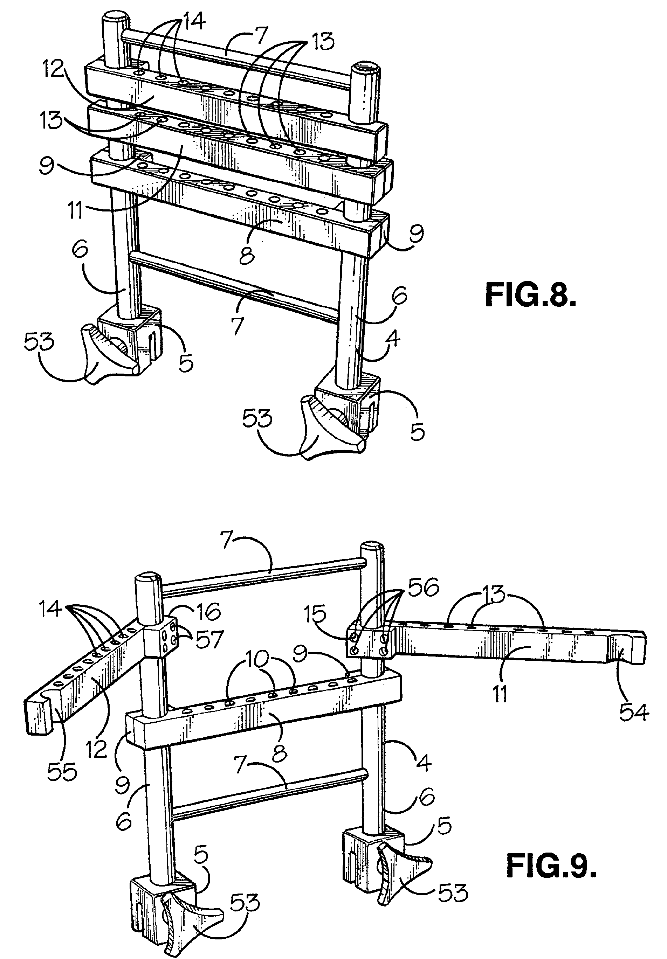

The frame 4, the main support member 8, and the pivotable arms 11, 12 are shown in greater detail in Figure 8 which is a view from the opposite side to that of Figure 1. Clamps 5 can be tightened on rail 2 by adjustment of knobs 53. As can be seen from Figure 9, pivotable arm 11 has a semi-cylindrical cutout portion 54 and pivotable arm 12 has a similar cutout 55 to facilitate folding the arms 11, 12 against frame 4. Clamp 15 is held in place so as to grip the corresponding vertical first member 6 by means of four screws 56, while similar screws 57 serve to enable clamp 16 to grip its corresponding vertical first member 6. Similar screws (not shown in Figure 1) are used to enable clamps 9 to secure main support member 8 in position on the vertical first members 6.

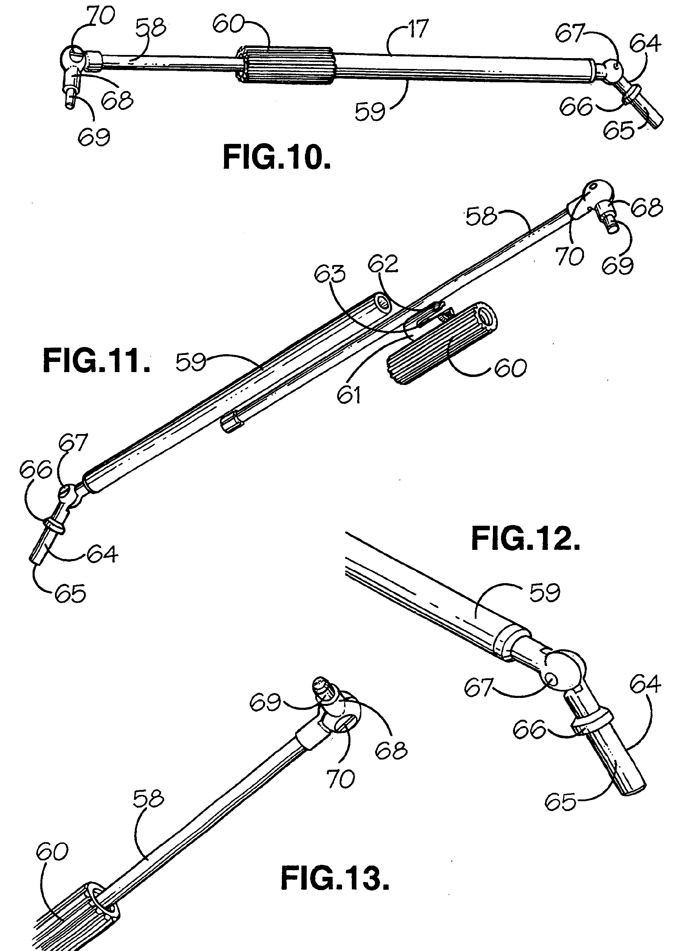

Figures 10 to 13 illustrate one of the telescopic cantilever members, for example first cantilever member 17. This comprises a rod 58 which is slidably received within a hollow outer member 59. The position of rod 58 relative to outer member 59 can be fixed by means of a rotatable locking member 60 which has an internal thread at its right hand end (as shown in Figure 10) so that it can be threadedly engaged with outer member 59 and which has a bore of smaller size at its left hand end (as shown in Figure 10) which snugly receives rod 58. As shown in Figure 11, a ferrule 61 is trapped on rod 58 by rotatable locking member 60. As locking member 60 is screwed on to outer member 59 so ferrule 61 is trapped by the right hand end (as shown in Figure 11) of locking member 60. Due to the chamfered end 62 of ferrule 61, a longitudinal slot 63 in ferrule 61 is caused to close thereby permitting ferrule 61 to clamp rod 58 relative to outer member 59 and so lock rod 58 firmly to hollow outer member 59 against extension or contraction and also against rotation of rod 58 relative to hollow outer member 59.

At its proximal end cantilever member 17 carries a short extension 64 on the free end of which is formed a spigot 65 for receipt in one of holes 10, 13, 14. Extension 64 also has a flange 66 to limit the extent to which spigot 65 can enter hole 10, 13 or 14. Extension 64 is pivotably attached to outer member 59 and its position relative thereto can be fixed by tightening screw 67.

The distal end of rod 58 carries a similar extension 68 with a spigot 69. Extension 68 is pivotably attached to rod 58 and its position relative thereto can be fixed by tightening screw 70 (see Figure 13).

5 The construction of the other cantilever members 18 to 22 is similar to that of cantilever member 17.

Just as spigot 65 is sized to fit snugly in one of the holes 10, 13, 14, so also spigot 69 is sized to fit snugly in one of holes 40, 45.

0 In use of the device of Figures 1 to 13, the operating. table 3 will be covered by a surgical drape (not shown). Frame 4 is then clamped on rail 2 over the surgical drape. When the surgeon is ready to clamp the patient's surgically exposed tibia T and femur F in position in readiness for use of the robot, he can adjust the height of support 8 and position arms 11, 12 in readiness for installation of the femur clamp 23 and the tibial attachment member 24. When the femur clamp 23 and tibial attachment member 24 have been positioned on the femur F and tibia T respectively, he can then support the femur clamp 23 by means of the three first cantilever members 17, 18, 19. To do this the spigots 65 are fitted in appropriate holes 10, 13, 14 with their locking members 60 loose and the lengths of the first cantilever members 17, 18, 19 adjusted so as to permit spigots 69 to fit in an appropriate one of holes 40. When the surgeon is satisfied with the positioning of first cantilever members 17, 18, 19 the locking members 60 can be tightened to fix the position of the patient's femur F. In a similar way second cantilever members 20, 21, 22 can be used to support the tibial attachment member 24. In this case the spigots 65 on the three second cantilever members 20, 21, 22 are fitted in appropriate holes 10, 13, 14 with their locking members 60 loose while the spigots 69 are fitted in appropriate holes 45 on tibial attachment member 24, the lengths of second cantilever members 20, 21 and 22 being adjusted as necessary before the locking members 60 are tightened. In this way the surgically exposed femur F and tibia T can be firmly clamped in position and prevented from movement during the subsequent stages of the knee prosthesis implantation operation.

Further support can be provided by means of the lower leg support 25 in conjunction with rack 26.

If desired the surgeon can first fix the position of the tibia T and then fix that of the femur F. Because of the design of the clamping device 1 it can be fitted so that essentially all of the clamping arrangement for the femur F and tibia T lies substantially at or below the operating area where the robot is to operate. With the tibia 7 and femur F now firmly clamped the surgeon can conduct registration so as to enable the robot subsequently to perform the desired bone cuts under the watchful eye of the surgeon.

When the bone cuts have been made, it then is a simple matter to loosen the locking members 60 on at least some of the cantilever members 17 to 22, whereupon the spigots 65, 69 can be withdrawn from the holes 10, 13, 14, 40, 45, the femur clamp 23 released and the screws 42 removed to allow tibial attachment member 24 also to be removed.

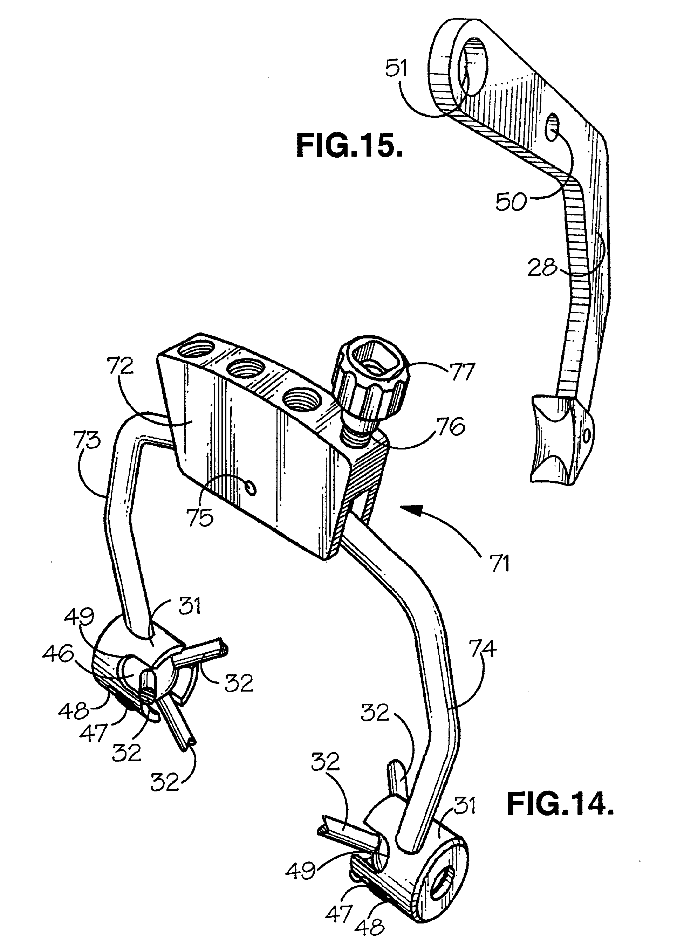

Figure 14 illustrates a modified form of femur clamp 71 which comprises a body 72 to which is welded a fixed gripping jaw 73. This carries at its free end a socket 31 provided with three prongs 32. A second movable gripping jaw 74 is pivotably mounted in body 72 so as to be pivotable about pivot 75 and also carries a socket 31 with three prongs 32. Movable gripping jaw 74 extends inside body 72 beyond pivot 75 (to the left as drawn) and a compression spring (not shown) within body 72 bears on a left hand end portion of jaw 74 so as to tend to move gripping jaw 74 towards the open position. An adjustment screw 76 with a knurled operating knob 77 is threadedly engaged in a bore in body 72 so that the lower end of screw 76 (as drawn) bears on the upper side of jaw 74. By screwing adjustment screw 76 further into body 72 jaw 74 can be made to move against the influence of the compression spring so as to close the jaws 73, 74 and cause the prongs 32 to contact and grip the patient's surgically exposed femur F.

In Figure 15 there is shown an alternative form of foot for use on femur clamp 23. This comprises a toggle foot pivotably attached to the jaw 28 by means of a pivot pin.

Instead of providing smooth ended spigots 65, 69 at both ends of cantilever members 17 to 22, either spigot 65 or spigot 69 (but not both) can be provided with a male screw thread, while the set of holes 11, 13, 14 or the set of holes 40, 45 (both not both sets of holes) is each formed with a corresponding female screw thread.

Alternatively one or both of spigots 65, 69 can be made in the form of expanding plugs operable by means of a knurled adjustment knob.

If desired, a movement detector can be incorporated in at least one of the cantilever members 17 to 22. Thus, for example, a movement detector can be incorporated in at least one of the first cantilever members 17, 18, 19 and in at least one of the second cantilever members 20, 12, 22. Such a movement detector or detectors would be arranged so as to result in a visual and/or audible alarm in the event of an accidental movement of the tibia T and/or the femur F being caused as a result, for example, of inadvertent disturbance thereof (e.g. by jogging of the patient or of the operating table by a member of the surgical team) or in the event of system failure.

Although the device 1 has been described as being suitable for attachment to one of the rails 2 extending along the side of operating table 3, it can alternatively be designed to be clamped to or otherwise supported by part of the robot or its housing.

It is further envisaged that the support for the operating head of the robot (i.e. the part that actually makes the saw cuts in the patient's femur F and tibia T) can be supported in appropriate position adjacent to the surgically exposed tibia T and femur F by a separate set of three cantilever members, similar to cantilever 17 to 19 or 20 to 22.

Instead of providing a femur clamp 23, it is also envisaged that femur clamp 23 can be replaced by a femoral attachment member adapted to be secured by screws to the femur F, in a similar way to that in which tibial attachment member 24 is fixed to tibia T as shown in the drawings. Alternatively tibial attachment member 24 can be replaced by a clamp similar to femoral clamp 23; this can be adapted for subcutaneous use and inserted through the skin or through a small incision penetrating to the bone.

Instead of providing three separate support members 8, 11, 12, it is alternatively possible to provide a single support member provided with holes 10, 13, 14 such that a central portion extends between first members 6 and wings extend outwardly from this central portion each at an oblique angle to the central portion so that they extend to either side of frame 4.

The device 1 has been illustrated in relation to use during a knee replacement operation. It can, however, alternatively be used with appropriate adaptation in any situation in which bone immobilisation is important.

Examples of such situations include:
1. Severe bone fracture (for healing).
2. Neck and back injuries.
3. X-rays and scans.
4. Laser treatment.
5. Radiotherapy.
6. Physical restraint.
7. Brain surgery.

## Claims

1. A device for immobilising bones of a patient requiring surgery or treatment, the device comprising:
substantially vertical frame means including a pair of substantially vertical first members and at least one second member extending between and bracing the first members;
support means movably mounted on at least one of the first members so as to permit adjustment of the height of the support means and arranged to be securable to the at least one first member;
a series of first cantilever members each having a proximal end and a distal end, each first cantilever member being adapted to be supported at the proximal end thereof by the support means and to extend in cantilever fashion therefrom;
first bone engagement means mounted at and supported by the distal ends of the first cantilever members for engagement with a surgically exposed first bone of a patient for securing the first bone in a desired position;
a series of second cantilever members each having a proximal end and a distal end, each second cantilever member being adapted to be supported at the proximal end thereof by the support means and to extend in cantilever fashion therefrom; and
second bone engagement means mounted at and supported by the distal ends of the second cantilever members for engagement with a surgically exposed second bone of the patient for securing the second bone in a desired position.

2. A device according to claim 1, in which the frame comprises a pair of second members which are integral with the first members and extend substantially horizontally therebetween.

3. A device according to claim 1 or claim 2, in which the support means comprises a main support member extending between the first members and adapted to be movable on and adjustably securable to the first members and at least one subsidiary support member adapted to be movable on and adjustably securable to a respective first member and to be pivotable with respect thereto in a substantially horizontal plane.

4. A device according to claim 3, in which the at least one support member further includes a pair of subsidiary support members each adapted to be movable on and pivotable in a substantially horizontal plane about a respective first member and to be adjustably securable thereto.

5. A device according to any one of claims 1 to 4, in which there are three first cantilever members.

6. A device according to claim 5, in which:
(i) the support means comprises:
a main support member extending between the first members and adapted to be vertically movable on and adjustably securable to the first members; and
a pair of subsidiary support members each adapted to be vertically movable on and pivotable in a substantially horizontal plane about a respective first member and to be adjustably securable thereto; and
(ii) each first cantilever member is supported at its proximal end by a respective one of the support members.

7. A device according to any one of claims 1 to 6, in which there are three second cantilever members.

8. A device according to claim 7, in which:
(i) the support means comprises:
a main support member extending between the first vertical members and adapted to be vertically movable on and adjustably securable to the first members; and
a pair of subsidiary support members each adapted to be vertically movable on and pivotable in a substantially horizontal plane about a respective first member and to be adjustably securable thereto; and
(ii) each second cantilever member is supported at its proximal end by a respective one of the support members.

9. A device according to any one of claims 1 to 8, in which the substantially vertical frame means is adapted for securement to a rail extending along one side of an operating table.

10. A device according to claim 9, in which each of the first members of the substantially vertical frame means is provided with a respective foot adapted to engage with the rail and to be lockable thereto.

11. A device according to any one of claims 1 to 10, in which the first bone engagement means is adapted to be engageable with a surgically exposed femur of the patient and in which the second bone engagement means is adapted to be engageable with a surgically exposed tibia of the patient.

12. A device according to claim 11, in which the first bone engagement means comprises a femoral gripping means comprising a pair of gripping jaw members pivoted one to another, each provided at its free end with femur contact means for contact with the femur, and adjustment means for closing the gripping jaws to cause the femur contact means to contact with and grip the femur and for opening the gripping jaws to release the femur.

13. A device according to claim 12, in which the femur contact means comprises a plurality of self adjusting feet for contact with the surgically exposed femur of the patient.

14. A device according to claim 12, in which the femur contact means comprises a toggle mounted foot for contact with the surgically exposed femur of the patient.

15. A device according to any one of claims 1 to 14, in which the second bone engagement means comprises a tibial attachment member adapted to be secured by means of pins or screws to a surgically exposed tibia of the patient.

16. A device according to any one of claims 1 to 15, in which the first cantilever members are adjustable in length.

17. A device according to any one of claims 1 to 16, in which the second cantilever members are adjustable in length.

18. A device according to any one of claims 1 to 17, in which the support means is provided with a plurality of downwardly extending vertical holes or recesses.

19. A device according to claim 18, in which the proximal end of each first cantilever member is provided with a spigot adapted for receipt in one of the vertical holes or recesses.

20. A device according to claim 18 or claim 19, in which the distal end of each first cantilever member is provided with a spigot adapted for engagement with the first bone engagement means.

21. A device according to any one of claims 18 to 20, in which the proximal end of each second cantilever member is provided with a spigot adapted for receipt in one of the vertical holes or recesses.

22. A device according to claim 21, in which the distal end of each second cantilever member is provided with a spigot adapted for engagement with the second bone engagement means.

23. A device according to any one of claims 1 to 12, in which the first and second cantilever members are each adjustable in length.

24. A device according to any one of claims 1 to 23, in which the first and second cantilever members each comprise an elongate outer tubular member and an elongate inner member adapted to slide telescopically in the outer tubular member, and locking means for locking the elongate inner member and the elongate outer tubular member one to another.

25. A device according to any one of claims 1 to 24, in which at least one cantilever member selected from the first cantilever members and the second cantilever members is provided with a movement detector.

26. A device according to claim 25, in which the movement detector is arranged to produce an alarm signal upon movement of the at least one cantilever member.
